# EUROPEAN PATENT APPLICATION

(11) **EP 2 388 621 A2**
(43) Date of publication of application: **23.11.2011**
(21) Application number: 10174649.3
(22) Date of filing: 31.08.2010
(51) Int. Cl.: G01T 1/16

(54) **Method for removing noise of PET signal using modeling in PET-MRI fusion device and PET system in PET-MRI fusion device using the same**

(30) Priority: 30.04.2010 KR 20100040516
(71) Applicant: Industrie University Cooperation Foundation Sogang University, Seoul 121-742 (KR)
(72) Inventor: Kang, Jihoon, Jeollanam-do 530-840 (KR); Choi, Yong, Seoul 138-913 (KR); Hu, Wei, Seoul 121-809 (KR); Huh, Yoon Suk, Seoul 140-200 (KR); Hong, Key Jo, Seoul 139-939 (KR); Lim, Hyun Keong, Seoul 121-854 (KR); Kim, Sangsu, Seoul 132-849 (KR)
(74) Representative: Grosse, Wolfgang

(57) **Abstract**

Provided is a method for removing noise of a positron emission tomography (PET) signal in a PET-magnetic resonance imaging (MRI) fusion device without using an MRI radio frequency (RF) shield that degrades image quality. The method includes: (a1) converting a PET analog signal into a digital signal having a predetermined sampling frequency; (b1) determining whether the resulting PET digital signal is to be included in image reconstruction based on modeling using sampling points of the PET digital signal or an integration value of the PET digital signal; and (c1) extracting only the PET digital signal that will be included in image reconstruction. The method allows acquisition of molecular-level images without declined performance of a PET detector.

## Description

### CROSS-REFERENCE TO RELATED APPLICATIONS

This application claims priority under 35 U.S.C. §119 to Korean Patent Application No. 10-2010-0040516, filed on April 30, 2010, in the Korean Intellectual Property Office, the disclosure of which is incorporated herein by reference in its entirety.

### TECHNICAL FIELD

The following disclosure relates to a method for processing a positron emission tomography (PET) signal in a PET-magnetic resonance imaging (MRI) fusion device, and a PET system in a PET-MRI fusion device using the same. More particularly, the following disclosure relates to a method for removing noise of a PET signal using modeling in a PET-MRI fusion device, and a PET system in a PET-MRI fusion device using the same.

### BACKGROUND

Medical imaging employed for cellular, pre-clinical and clinical tests and diagnosis of patients is largely classified into structural imaging and functional imaging. Structural imaging refers to structural and anatomical of the human body, and functional imaging refers to imaging of functional information of the cognitive, sensual or other functions of the human body in a direct or indirect manner. The structural or anatomical imaging technique includes computed tomography (CT), magnetic resonance imaging (MRI), or the like. For imaging of functional information about the human body's physiological and biochemical functions, positron emission tomography (PET) is widely used.

PET is a powerful biological imaging tool allowing monitoring of the functional processes of the human body in a noninvasive manner. A biological probe molecule labeled with a radioactive, positron-emitting isotope is injected into the body, and the distribution of radiation is reconstructed through tomography to visualize and quantify the physiological and biochemical responses in the body organs. The functional and molecular biological information about the brain or other organs provided by PET may be useful for the etiological study of disease, diagnosis, prognosis, monitoring after anticancer treatment, or the like.

In order to provide functional information of the human body tissues with a high sensitivity of molecular level and to overcome the problem of low resolution, PET fusion medical imaging devices such as PET-CT, PET-MRI and PET-optical imaging are being developed.

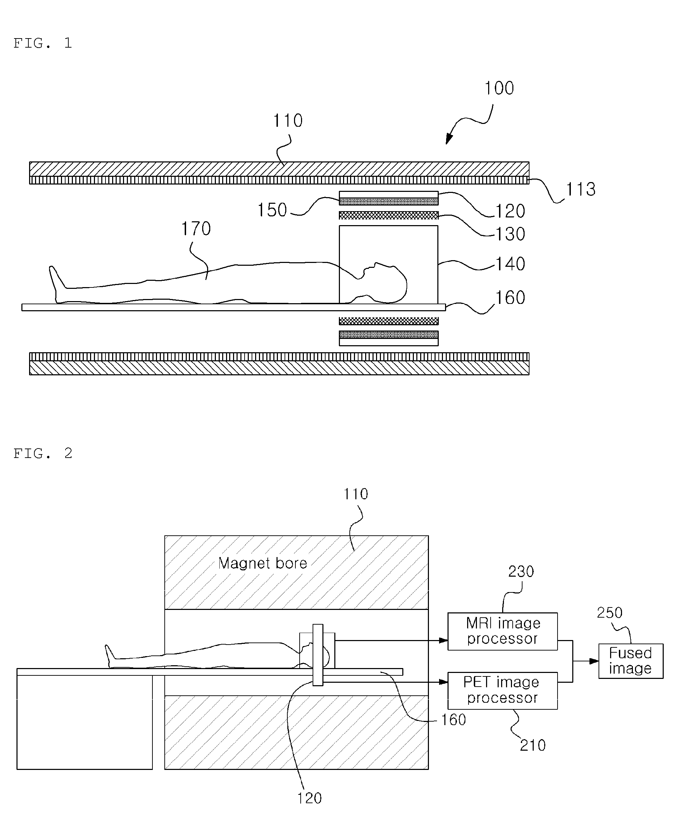

FIG. 1 is a cross-sectional view of an existing PET-MRI fusion device.

Referring to FIG. 1, a PET-MRI fusion device 100 includes a magnet bore 110, a PET detector 120, a radio frequency (RF) receiving coil 130, an RF transmitting coil 140, an RF shield 150 and a bed 160.

The PET detector 120 is provided between the RF receiving coil 130 and the magnet bore 110. The configuration of the PET-MRI fusion device 100 induces interaction between PET and MRI. As a result, various noises that deteriorate image quality are produced. The noises include electromagnetic interference, high-frequency and low-frequency interferences, etc. caused by MRI, and magnetic field distortion,decreasedsignal-to-noise ratio,eddy current, etc. caused by PET. Especially, since the high-magnetic-field, high-frequency energy of MRI has the largest effect on the acquisition of PET signals and the reconstruction of images, the RF shield 150 is provided between the RF receiving coil 130 and the PET detector 120 to minimize the interference. The RF shield 150 may comprise copper (Cu). However, the RF shield 150, which is typically in the form of a conductive cylinder, reduces performance of the RF receiving/transmitting coils 120, 140 of MRI, and degrades resolution of MRI images because of eddy current occurring in a gradient coil 113. Further, since the PET detector 120 is provided outside the RF receiving coil 130 with respect to the center of the magnet bore 110, gamma rays emitted from a subject 170 may be attenuated and diffused by the RF receiving coil 130, thereby resulting in declined PET signal detection ability. Moreover, heating of the PET detector 120 may result in declined performance. Further, the RF shield 150 provided to minimize the high-magnetic-field, high-frequency energy of MRI results in degraded image quality of the PET-MRI fusion device 100.

### SUMMARY

The present disclosure is directed to providing a method for removing noise of a positron emission tomography (PET) signal in a PET-magnetic resonance imaging (MRI) fusion device without using an MRI radio frequency (RF) shield that degrades image quality of the PET-MRI fusion device, and a PET system in a PET-MRI fusion capable of removing noise of a PET signal in a PET-MRI fusion device.

In one general aspect, the present disclosure provides a method for removing noise of a PET signal in a PET-MRI fusion device, including: (a1) converting a PET analog signal into a digital signal having a predetermined sampling frequency; (b1) determining whether the resulting PET digital signal is to be included in image reconstruction based on modeling using sampling points of the PET digital signal or an integration value of the PET digital signal; and (c1) extracting only the PET digital signal that will be included in image reconstruction.

In the modeling of the step (b1), when point a-1 is preceded by point a-2 among sampling points of the PET digital signal with constant time intervals, if an absolute value of the difference of voltages of the points v(a-2) and v(a-1) is within a predetermined range, the PET digital signal may be determined to be included in image reconstruction.

In the modeling of the step (b1), when point a+1 is preceded by point a among sampling points of the PET digital signal with constant time intervals, if an absolute value of the difference of voltages of the points v(a) and v(a+1) is within a predetermined range, the PET digital signal may be determined to be included in image reconstruction.

In the modeling of the step (b1), if the difference of voltages of points of maximum rising of the PET digital signal is within a predetermined range, the PET digital signal may be determined to be included in image reconstruction.

In the modeling of the step (b1), if an integration value of the PET digital signal is within a predetermined range, the PET digital signal may be determined to be included in image reconstruction.

In the modeling of the step (b1), if a maximum voltage extracted from the PET digital signal is within a predetermined range, the PET digital signal may be determined to be included in image reconstruction.

In another general aspect, the present disclosure provides a recording medium capable of being read by a digital processor and recording a program of commands which may be executed by the digital processor to implement the method for removing noise of a PET signal in a PET-MRI fusion device.

In another general aspect, the present disclosure provides a PET system in a PET-MRI fusion device, including: a PET detector detecting gamma rays emitted from a subject and converting flash light changed from the gamma rays into an electrical analog signal; a signal amplification unit amplifying the electrical analog signal input from the PET detector; a data acquisition unit converting the amplified electrical analog signal into a digital signal; and a signal modeling/processing unit determining whether the PET digital signal is to be included in image reconstruction based on the above modeling, extracting the signal and processing the signal for image reconstruction.

In another general aspect, the present disclosure provides a method for removing noise of a PET signal in a PET-MRI fusion device, including: (a2) measuring in real time a current required for amplifying an analog signal input from a PET detector by a signal amplification unit; (b2) determining whether the rise of the required current is larger than a predetermined critical value; and (c2) if the rise of the required current is larger than a predetermined critical value, controlling a voltage applied to amplify the signal.

In the step (c2), the voltage applied to amplify the signal may be decreased to reduce amplification factor.

In another general aspect, the present disclosure provides a PET system in a PET-MRI fusion device, including: a PET detector detecting gamma rays emitted from a subject and converting flash light changed from the gamma rays into an electrical analog signal; a signal amplification unit amplifying the electrical analog signal input from the PET detector; and a constant current control unit measuring in real time a current required by the signal amplification unit and controlling a voltage applied to amplify the signal if the rise of the required current is larger than a predetermined critical value.

The PET system in a PET-MRI fusion device may further include a data acquisition unit converting the amplified electrical analog signal into a digital signal.

The PET system in a PET-MRI fusion device may further include a signal processing unit processing the digital signal for image reconstruction.

The method for removing noise of a PET signal using modeling in a PET-MRI fusion device and the PET system in a PET-MRI fusion device according to the disclosure allow acquisition of molecular-level images in MRI environment without declined performance of the PET detector. Decline of performance such as PET non-response time, decreased sensitivity, distortion of images, etc. is minimized. In addition, the method for removing noise of a PET signal using modeling in a PET-MRI fusion device according to the disclosure and the PET system in a PET-MRI fusion device using the same can solve the problem of mutual interference associated with the existing method and system using an RF shield. Further, the presently disclosed system allows easy and simple setup.

Other features and aspects will be apparent from the following detailed description, the drawings, and the claims.

### BRIEF DESCRIPTION OF THE DRAWINGS

The above and other objects, features and advantages of the present disclosure will become apparent from the following description of certain exemplary embodiments given in conjunction with the accompanying drawings, in which:
FIG. 1 is a cross-sectional view of an existing positron emission tomography (PET)-magnetic resonance imaging (MRI) fusion device;
FIG. 2 illustrates a PET-MRI fusion device according to an embodiment;
FIG. 3 is a graph showing a PET detector output signal without an effect of noise;
FIG. 4 is a graph showing a PET detector output signal affected by noise of high-magnetic-field, high-frequency energy of MRI in a PET-MRI fusion device;
FIG. 5 is a flow chart illustrating a method for removing noise of a PET signal using modeling in a PET-MRI fusion device according to an embodiment;
FIG. 6 shows examples of acquiring sampling points from a PET digital signal during rising time and falling time;
FIG. 7 shows an example wherein a pulse signal is determined not to be included in image reconstruction according to a first modeling method;
FIG. 8 shows an example wherein a pulse signal is determined not to be included in image reconstruction according to a secondmodeling method;
FIG. 9 shows an example wherein a pulse signal is determined to be included in image reconstruction according to a fourth modeling method, and FIG. 10 shows an example wherein a pulse signal is determined not to be included in image reconstruction according to the fourth modeling method;
FIG. 11 is a block diagram illustrating a PET system in a PET-MRI fusion device according to an embodiment;
FIG. 12 is a flow chart illustrating a method for removing noise of a PET signal in a PET-MRI fusion device according to another embodiment;
FIG. 13 is a block diagram illustrating a PET system in a PET-MRI fusion device according to another embodiment; and
FIG. 14 is a graph showing an energy spectrum after removal of noise of a PET signal in a PET-MRI fusion device according to an embodiment.

It should be understood that the appended drawings are not necessarily to scale, presenting a somewhat simplified representation of various preferred features illustrative of the basic principles of the disclosure. The specific design features of the disclosure as disclosed herein, including, for example, specific dimensions, orientations, locations and shapes, will be determined in part by the particular intended application and use environment.

In the figures, reference numerals refer to the same or equivalent parts of the disclosure throughout the several figures of the drawings.

### DETAILED DESCRIPTION OF EMBODIMENTS

Hereinafter, description will be made about particular embodiments of the present disclosure which may be variously modified. However, it is not intended to limit the present disclosure to specific embodiments. On the contrary, the present disclosure is intended to cover not only the exemplary equivalents but also various alternatives, modifications, equivalents and other equivalents that may be included within the spirit and scope the present disclosure are.

While terms including ordinal numbers, such as "first", "second", etc., may be used to describe various components, such components are not limited to the above terms. Those terms are used only to distinguish one component from another. For example, without departing from the scope of the present disclosure, a first component may be referred to as a second component, and likewise a second component may be referred to as a first component. The term and/or encompasses both combinations of the plurality of related items disclosed and any one item from among the plurality of related items disclosed.

When a component is mentioned to be "connected" to or "accessing" another component, this may mean that it is directly connected to or accessing the other component, but it is to be understood that another component may exist in between. On the other end, when a component is mentioned to be "directly connected" to or "directly accessing" another component, it is to be understood that there are no other components in between.

The terms used in the present specification are merely used to describe particular embodiments, and are not intended to limit the present disclosure. An expression used in the singular encompasses the expression of the plural, unless it has a clearly different meaning in the context. In the present specification, it is to be understood that the terms such as "including", "having", etc. are intended to indicate the existence of the features, numbers, operations, components, parts or combinations thereof disclosed in the specification, and are not intended to preclude the possibility that one or more other features, numbers, operations, components, parts or combinations thereof may exist or may be added.

Unless otherwise defined, all terms used herein, including technical and scientific terms, have the same meanings as those generally understood by those with ordinary knowledge in the field of art to which the present disclosure belongs. Such terms as those defined in a generally used dictionary are to be interpreted to have the meanings equal to the contextual meanings in the relevant filed of art, and are not to be interpreted to have ideal or excessively formal meanings unless clearly defined in the present specification.

Certain embodiments of the present disclosure will be described below in more detail with reference to the accompanying drawings, in which those components are rendered the same reference numeral that are the same or are in correspondence, regardless of the figure number, and redundant explanations are omitted.

FIG. 2 illustrates a positron emission tomography (PET)-magnetic resonance imaging (MRI) fusion device according to an embodiment.

Referring to FIG. 2, a PET system comprising a PET detector 120 and a PET image processor 210 removes noise of a PET signal in a PET-MRI fusion device. The MRI operates independently.

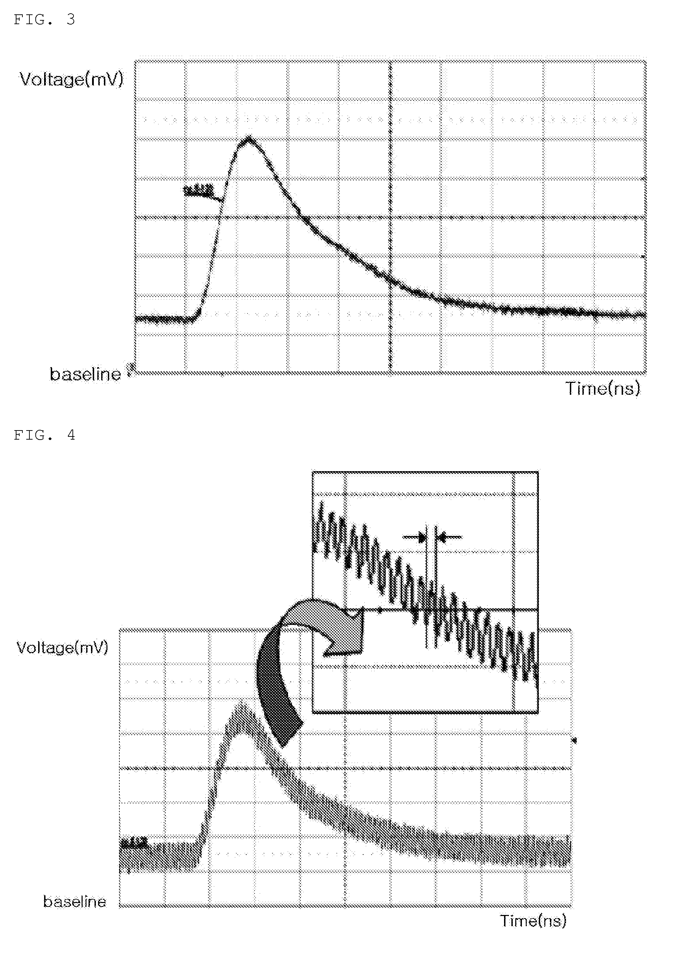

FIG. 3 is a graph showing a PET detector output signal without an effect of noise.

FIG. 4 is a graph showing a PET detector output signal affected by noise of high-magnetic-field, high-frequency energy of MRI in a PET-MRI fusion device. Referring to FIG. 4, the PET signal is not clear because of noise.

A method for removing noise of a PET signal using modeling in a PET-MRI fusion device according to an embodiment of the disclosure utilizes the fact that a PET signal with noise included has different characteristics from a normal PET signal.

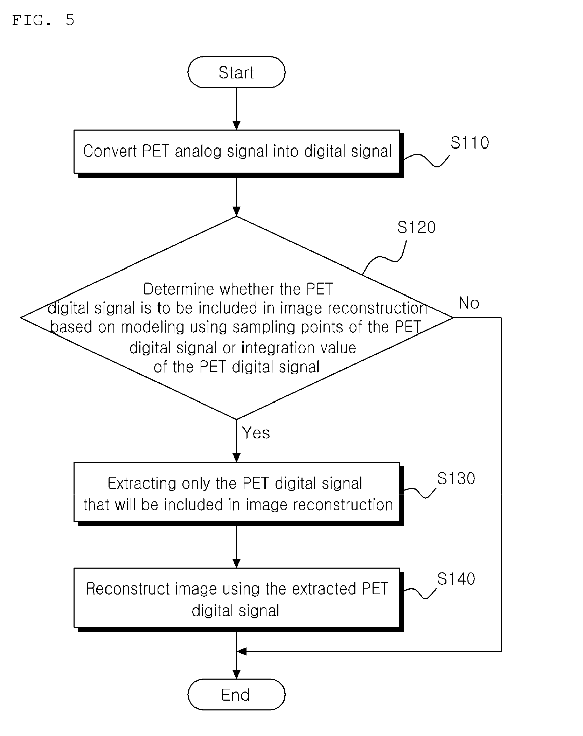

FIG. 5 is a flow chart illustrating a method for removing noise of a PET signal using modeling in a PET-MRI fusion device according to an embodiment.

Referring to FIG. 5, an electrical analog signal from a PET detector is converted into a digital signal using an analog-to-digital converter (ADC) (S110).

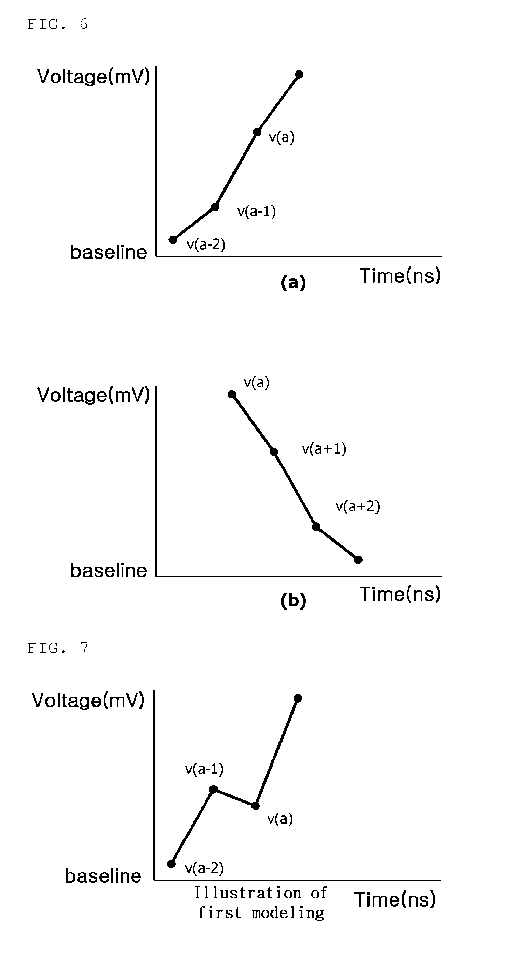

FIG. 6 shows examples of acquiring sampling points from a PET digital signal during rising time and falling time.

Referring to FIG. 6 (a), point a-2, point a-1 and point a are points at 10 ns intervals of a gamma ray pulse signal sampled and digitized during rising time. The time interval between the points a-2, a-1 and a may be changed depending on the operation frequency of the ADC. Those skilled in the art will understand that the number of the points may be changed variously. v(a-2), v(a-1) and v(a) are voltages at the respective points.

Referring to FIG. 6 (b), point a, point a+1 and point a+2 are points at 10 ns intervals of a gamma ray pulse signal sampled and digitized during falling time.

The specific numbers used in the description are given only to describe the embodiments of the present disclosure, and the present disclosure is not limited by those specific numbers. Accordingly, those skilled in the art will understand that the time interval 10 ns may be changed differently.

Then, it is determined whether the PET digital signal is to be included in image reconstruction based on modeling using the sampling points of the PET digital signal or an integration value of the PET digital signal (S120).

Hereinafter, a modeling method for determining whether a pulse signal is to be included in image reconstruction will be described.

Referring to FIG. 6 (a), in a normal signal with no noise, the relationship v(a-2) < v(a-1) < v(a) will be satisfied. If an absolute value of the difference of v(a-2) and v(a-1) or an absolute value of the difference of v(a-1) and v(a) is within a predetermined range, the PET digital signal is determined to be included in image reconstruction [first modeling]. Here, the points a-2, a-1 and a are points at 10 ns intervals of a gamma ray pulse signal sampled and digitized during rising time. Those skilled in the art will understand that the number of the points may be changed variously. For example, if the predetermined range is 0.2 to 0.5 mV and the absolute value of the difference of v(a-2) and v(a-1) is 0.1 or 0.7mV, the PET digital signal is determined not to be included in image reconstruction. Otherwise, if the absolute value is 0.3 mV, the PET digital signal is determined to be included in image reconstruction. The predetermined range may be set through a statistical procedure for a plurality of PET signals unaffected by noise.

FIG. 7 shows an example wherein a pulse signal is determined not to be included in image reconstruction according to the first modeling method.

Referring to FIG. 6 (b), in a normal signal with no noise, the relationship v(a) < v(a+1) < V(a+2) will be satisfied. If an absolute value of the difference of v(a) and v(a+1) or an absolute value of the difference of v(a+1) and v(a+2) is within a predetermined range, the PET digital signal is determined to be included in image reconstruction [second modeling]. Here, the points a, a+1 and a+2 are points at 10 ns intervals of a gamma ray pulse signal sampled and digitized during falling time. Those skilled in the art will understand that the number of the points may be changed variously. The predetermined range may be set through a statistical procedure for a plurality of PET signals unaffected by noise.

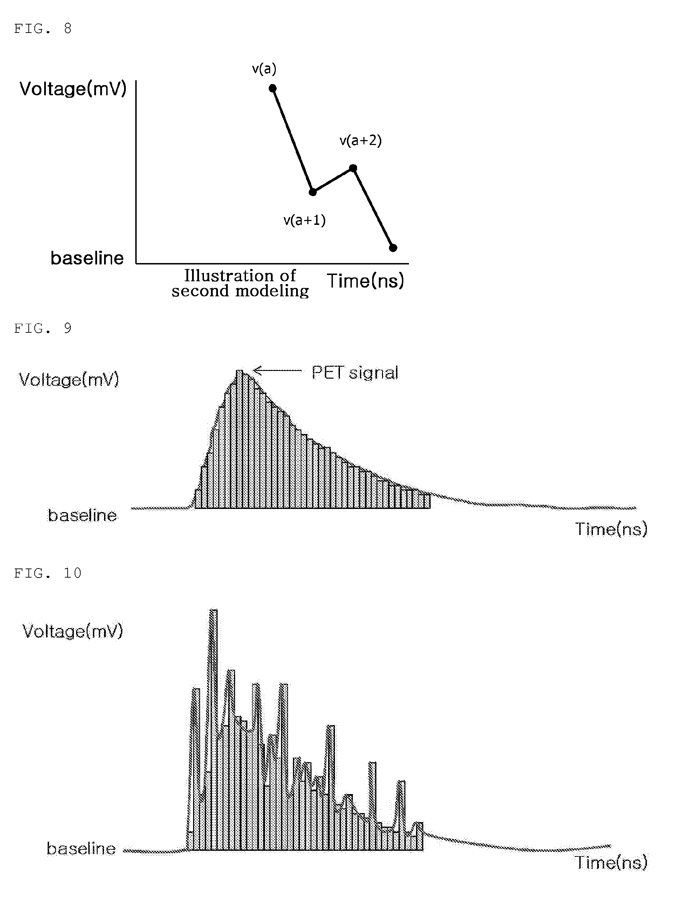

FIG. 8 shows an example wherein a pulse signal is determined not to be included in image reconstruction according to the second modeling method.

As an alternative modeling method, if the difference of voltages of points of maximum rising of the PET digital signal is within a predetermined range, the PET digital signal is determined to be included in image reconstruction [third modeling method]. For example, if the difference of voltages of points of maximum rising is larger than 15 mV and smaller than 150 mV, the PET digital signal may be determined to be included in image reconstruction.

As another alternative modeling method, if an integration value of the PET digital signal is within a predetermined range, the PET digital signal is determined to be included in image reconstruction [fourth modeling method]. For example, if the integration value of the PET digital signal is within 1000 and 1275 keV, the PET digital signal may be determined to be included in image reconstruction.

FIG. 9 shows an example wherein a pulse signal is determined to be included in image reconstruction according to the fourth modeling method, and FIG. 10 shows an example wherein a pulse signal is determined not to be included in image reconstruction according to the fourth modeling method.

As another modeling method, if a maximum voltage extracted from the PET digital signal is within a predetermined range, the PET digital signal may be determined to be included in image reconstruction [fifth modeling].

In a method for removing noise of a PET signal in a PET-MRI fusion device according to the disclosure, various other modeling methods based on the fact that a PET signal with noise included has different characteristics from a normal PET signal may be utilized.

Referring again to FIG. 5, only the PET digital signal that will be included in image reconstruction is extracted (S130). Then, an image is reconstructed using the extracted PET digital signal (S140).

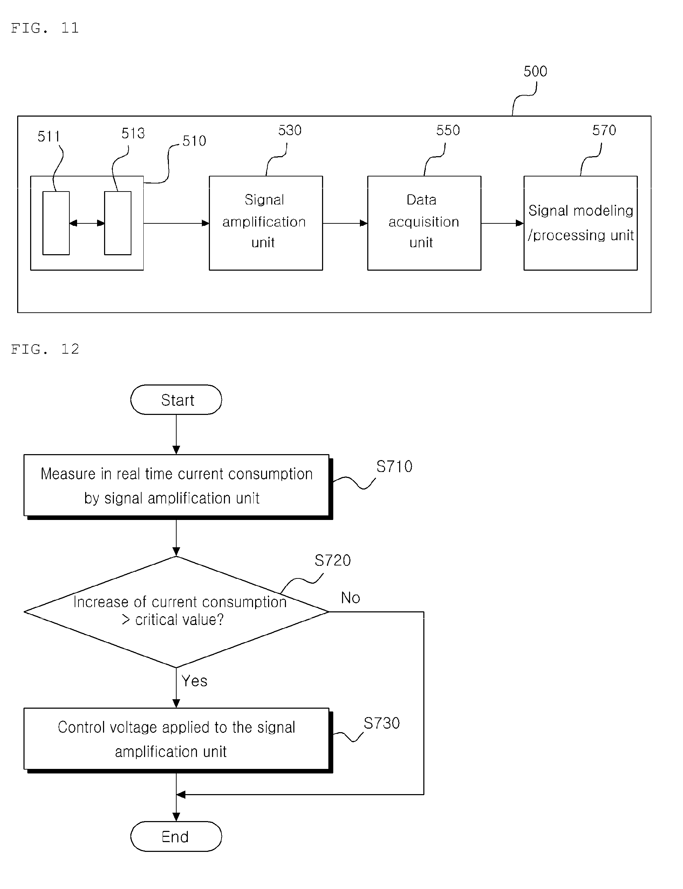

FIG. 11 is a block diagram illustrating a PET system in a PET-MRI fusion device according to an embodiment.

Referring to FIG. 11, a PET system 500 in a PET-MRI fusion device comprises a PET detector 510, a signal amplification unit 530, a data acquisition unit 550 and a signal modeling/processing unit 570.

The PET detector 510 comprises a scintillation crystal 511 and a photosensor 513. The scintillation crystal 511 detects gamma rays emitted from a subject and converts them into a flash light. For example, the scintillation crystal 511 detects 511 keV gamma rays emitted through a pair annihilation phenomenon toward opposite directions. The scintillation crystal 511 may be selected frombismuth germanate (BGO), lutetium oxyorthosilicate (LSO), lutetium yttrium oxyorthosilicate (LYSO), lutetium aluminum perovskite (LuAP), lutetium yttrium aluminum perovskite (LuYAP), lanthanumbromide (LaBr₃), lutetium iodide (LuI₃), gadolinium oxyorthosilicate (GSO), lutetium gadolinium oxyorthosilicate (LGSO) and lutetium aluminum garnet (LuAG). The photosensor 513 may be a photomultiplier tube (PMT), a positive-intrinsic-negative PIN) diode, cadmium telluride (CdTe), cadmium zinc telluride (CZT), an avalanche photodiode (APD), a Geiger-mode avalanche photodiode (GAPD), or the like.

The signal amplification unit 530 amplifies weak electrical signals input from the PET detector 510 and increases number of signal channels to enable data acquisition and signal processing. The data acquisition unit 550 converts the electrical analog signal into a PET digital signal to allow signal processing and image reconstruction. The signal modeling/processing unit 570 determines whether the PET digital signal is to be included in image reconstruction based on the above modeling, extracts the signal and processes the signal for image reconstruction. Here, one or more modeling method (s) selected from the afore-described first through fifth modeling methods may be utilized.

FIG. 12 is a flow chart illustrating a method for removing noise of a PET signal in a PET-MRI fusion device according to another embodiment. A procedure for acquisition of an MRI image in a PET-MRI fusion device may be largely divided into a radio frequency (RF) signal excitation period and an RF signal acquisition period. Of the two, the RF signal excitation period has a larger effect on the PET signal. During the operation of the PET-MRI fusion device, RF noise affects the processing of the PET signal. The RF noise exerts the largest effect on the PET signal processing when the PET signal is affected by magnetic resonance (MR). In that case, current consumption by the signal amplification unit increases rapidly. Another embodiment of the present disclosure is based on this phenomenon.

Referring to FIG. 12, the current required for amplifying a weak electrical signal input from a PET detector by a signal amplification unit is measured in real time (S710).

If the rise of the required current is larger than a predetermined critical value (S720), a voltage applied to the signal amplification unit is controlled (S730). Specifically, the voltage applied to the signal amplification unit may be decreased. For example, assume a 5V, 200 mA consumption for a normal PET signal, with an amplification factor of 1000. Then, the PET signal has a magnitude of 1V. If the rise of current consumption for processing the PET signal with RF noise is large, the voltage applied to the signal amplification unit may be decreased to 4 V so that the current consumption is decreased to 250 mA. Then, the amplification factor becomes 800, and the PET signal has a magnitude of 0.8 V. If the period where the rise of the required current is larger than the critical value changes periodically, the amplification factor of the signal amplification unit may be programmed to be varied periodically. That is to say, the noise of the PET signal in the PET-MRI fusion device is removed by varying the amplification factor of the signal amplification unit.

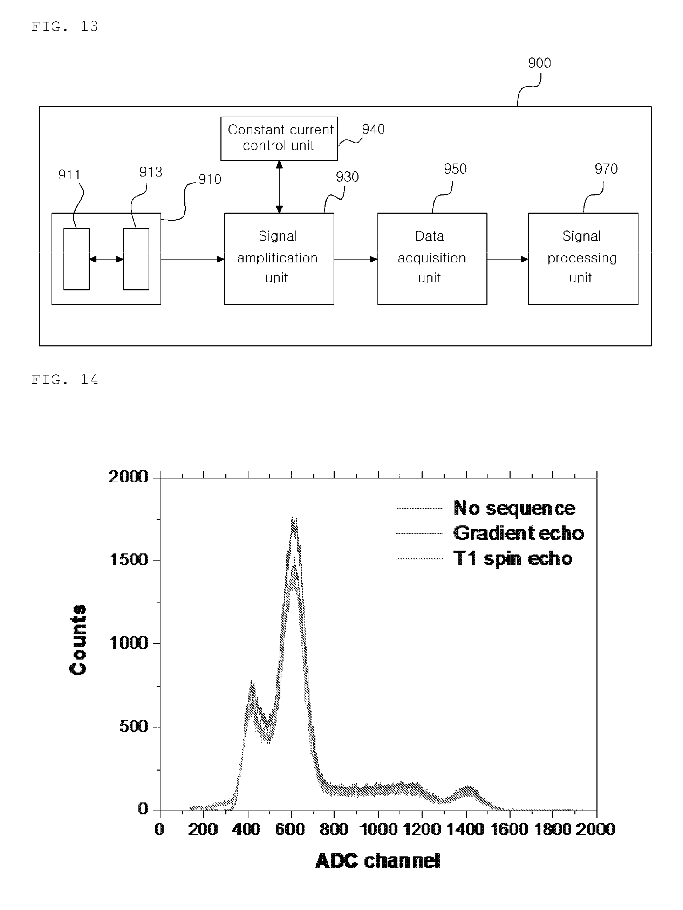

FIG. 13 is a block diagram illustrating a PET system in a PET-MRI fusion device according to another embodiment.

Referring to FIG. 13, a PET system 900 in a PET-MRI fusion device comprises a PET detector 910, a signal amplificationunit 930, a constant current control unit 940, a data acquisition unit 950 and a signal processing unit 970.

The PET detector 910 comprises a scintillation crystal 911 and a photosensor 913. It detects gamma rays emitted from a subject and converting flash light changed from the gamma rays into an electrical analog signal. The signal amplification unit 930 amplifies the weak electrical signal input from the PET detector 910 and increases number of signal channels to enable data acquisition and signal processing. The amplification factor of the signal amplification unit 930 is not constant but is decreased when the RF noise has a large effect on the PET signal processing. The constant current control unit 940 measures in real time a current required by the signal amplification unit 930 and controls a voltage applied to the signal amplification unit 930 if the rise of the required current is larger than a predetermined critical value. Specifically, the voltage applied to the signal amplification unit 930 may be decreased. The data acquisition unit 950 converts the electrical analog signal into a PET digital signal through sampling. The signal processing unit 970 processes the PET digital signal for image reconstruction.

A method for removing noise of a PET signal using modeling in a PET-MRI fusion device according to an embodiment of the disclosure may be used at the same time with a method for removing noise of a PET signal in a PET-MRI fusion device according to another embodiment of the disclosure. And, the feature of a PET system in a PET-MRI fusion device according to an embodiment of the disclosure may exist together with the feature of a PET system in a PET-MRI fusion device according to another embodiment of the disclosure.

FIG. 14 is a graph showing an energy spectrum after removal of noise of a PET signal in a PET-MRI fusion device according to an embodiment. Referring to FIG. 14, it can be seen that the noise was removed.

The term "unit" used in the specification refers to, but is not limited to, a software or hardware component, such as a field-programmable gate array (FPGA) or an application-specific integrated circuit (ASIC), which executes certain tasks. A unit may be configured to reside in the addressable storage medium, and configured to execute on one or more processors. Thus, a unit may include, by way of example, components, such as software components, object-oriented software components, class components and task components, processes, functions, attributes, procedures, subroutines, segments of program code, drivers, firmware, microcode, circuitry, data, databases, databases structures, tables, arrays and variables. The functionality provided for in the components and units may be combined into fewer components and units or further separated into additional components and units. In addition, the components and units may be implemented such that they execute one or more CPU(s) in a device or a secure multimedia card.

The specific numbers used in the above embodiments are given only to describe the embodiments of the present disclosure, and the present disclosure is not limited by those specific numbers.

The functionalities described above may be implemented by a processor such as a microprocessor, a controller, a microcontroller, an ASIC, etc. according to software or program codes coded to execute such functionalities. Designing, development and implementation of such codes will be easily understood by those skilled in the art based on the description of the present disclosure.

For example, the program may be recorded on a hard disk or in a read-only memory (ROM) as a recording medium in advance. Alternatively, the program may be temporarily or permanently stored on a removable recording medium such as a flexible disk, a compact disc read-only memory (CD-ROM), a magneto-optical (MO) disk, a digital versatile disc (DVD), a magnetic disk or a semiconductor memory. Such a removable recording medium can be provided as package software.

While the present disclosure has been described with respect to the specific embodiments, it will be apparent to those skilled in the art that various changes and modifications may be made without departing from the spirit and scope of the disclosure as defined in the following claims.

## Claims

1. A method for removing noise of a positron emission tomography (PET) signal in a PET-magnetic resonance imaging (MRI) fusion device, comprising:
converting a PET analog signal into a digital signal having a predetermined sampling frequency;
determining whether the resulting PET digital signal is to be included in image reconstruction based on modeling using sampling points of the PET digital signal or an integration value of the PET digital signal; and
extracting only the PET digital signal that will be included in image reconstruction.

2. The method for removing noise of a PET signal in a PET-MRI fusion device according to claim 1, wherein, in the modeling, when point a-1 is preceded by point a-2 among sampling points of the PET digital signal with constant time intervals, if an absolute value of the difference of voltages of the points v(a-2) and v(a-1) is within a predetermined range, the PET digital signal is determined to be included in image reconstruction.

3. The method for removing noise of a PET signal in a PET-MRI fusion device according to claim 1, wherein, in the modeling, when point a+1 is preceded by point a among sampling points of the PET digital signal with constant time intervals, if an absolute value of the difference of voltages of the points v(a) and v(a+1) is within a predetermined range, the PET digital signal is determined to be included in image reconstruction.

4. The method for removing noise of a PET signal in a PET-MRI fusion device according to claim 1, wherein, in the modeling, if the difference of voltages of points of maximum rising of the PET digital signal is within a predetermined range, the PET digital signal is determined to be included in image reconstruction.

5. The method for removing noise of a PET signal in a PET-MRI fusion device according to claim 1, wherein, in the modeling, if an integration value of the PET digital signal is within a predetermined range, the PET digital signal is determined to be included in image reconstruction.

6. The method for removing noise of a PET signal in a PET-MRI fusion device according to claim 1, wherein, in the modeling, if a maximum voltage extracted from the PET digital signal is within a predetermined range, the PET digital signal is determined to be included in image reconstruction.

7. A recording medium capable of being read by a digital processor and recording a program of commands which may be executed by the digital processor to implement the method for removing noise of a PET signal in a PET-MRI fusion device according to any one of claims 1 to 6.

8. A positron emission tomography (PET) system in a PET-magnetic resonance imaging (MRI) fusion device, comprising:
a PET detector detecting gamma rays emitted from a subject and converting flash light changed from the gamma rays into an electrical analog signal;
a signal amplification unit amplifying the electrical analog signal input from the PET detector;
a data acquisition unit converting the amplified electrical analog signal into a digital signal; and
a signal modeling/processing unit determining whether the PET digital signal is to be included in image reconstruction based on the modeling according to according to any one of claims 1 to 6, extracting the signal and processing the signal for image reconstruction.

9. A method for removing noise of a positron emission tomography (PET) signal in a PET-magnetic resonance imaging (MRI) fusion device, comprising:
measuring in real time a current required for amplifying an analog signal input from a PET detector by a signal amplification unit;
determining whether the rise of the required current is larger than a predetermined critical value; and
if the rise of the required current is larger than a predetermined critical value, controlling a voltage applied to amplify the signal.

10. The method for removing noise of a PET signal in a PET-MRI fusion device according to claim 9, wherein, in said controlling the voltage, the voltage applied to amplify the signal is decreased to reduce amplification factor.

11. A recording medium capable of being read by a digital processor and recording a program of commands which may be executed by the digital processor to implement the method for removing noise of a PET signal in a PET-MRI fusion device according to claim 9 or 10.

12. A positron emission tomography (PET) system in a PET-magnetic resonance imaging (MRI) fusion device, comprising:
a PET detector detecting gamma rays emitted from a subject and converting flash light changed from the gamma rays into an electrical analog signal;
a signal amplification unit amplifying the electrical analog signal input from the PET detector; and
a constant current control unit measuring in real time a current required by the signal amplification unit and controlling a voltage applied to amplify the signal if the rise of the required current is larger than a predetermined critical value.

13. The PET system in a PET-MRI fusion device according to claim 12, which further comprises a data acquisition unit converting the amplified electrical analog signal into a digital signal.

14. The PET system in a PET-MRI fusion device according to claim 13, which further comprises a signal processing unit processing the digital signal for image reconstruction.
